# EUROPEAN PATENT APPLICATION

(11) **EP 1 774 930 A1**
(43) Date of publication of application: **18.04.2007**
(21) Application number: 06122198.2
(22) Date of filing: 12.10.2006
(51) Int. Cl.: A61F 5/01, A63B 26/00, A63B 22/16

(54) **Board for proprioceptive rehabilitation of the upper and lower limbs**

(30) Priority: 17.10.2005 IT VR20050130
(71) Applicant: F.G.P. Srl, 37062 Dossobuono (VR) (IT)
(72) Inventor: Ferrigolo, Moreno, 37062, Dossobuono (VR) (IT); Turrini, Alberto, 37060, Castel D'Azzano (VR) (IT)
(74) Representative: Petraz, Gilberto Luigi

(57) **Abstract**

A proprioceptive board (10) for the rehabilitation of joints, in particular for the treatment of the upper and/or lower limbs, consisting of a support surface (11) which is rectangular or another similar elongated shape to allow the part of the limb to be rehabilitated to be rested on it; the lower side of the support surface (11) is fitted with a roller (12), which allows the support surface to rock according to certain angulations, the roller (12) being fitted on the lower side of the support surface by means of an interposed plate (13) hinged at its centre (14) to the centre of the board (11). The board is also equipped with an orientation aid (16) positioned on the lower side of the support surface.

## Description

### TECHNICAL FIELD

This invention concerns a proprioceptive board for the rehabilitation of limbs, in particular for treatment of the ankle, the shoulder, the knee, the elbow, the hip or other joints of the upper or lower limbs.

More specifically, this invention refers to a proprioceptive board designed in such a way as to allow the rehabilitation of limbs and joints of subjects who have suffered injury to the joints.

The board according to the invention represents a simple but extremely effective means of rehabilitation for certain body joints, with the advantage over known solutions of being able to use a very inexpensive means which is at the same time extremely versatile for this type of orthopedic use.

This invention can be applied in the production sector of orthopedic instruments and accessories in general but also of prostheses and braces mainly used in conservative, post-traumatic and post-operative therapy.

### BACKGROUND ART

It is known that with some diseases or in the case of some orthopedic type problems, for example following a sprain to the ankle or the shoulder, it is necessary after clinical healing for the subject to re-acquire full ability to carry out all the movements of the injured joint once again, using rehabilitation means and methods.

If correct rehabilitation is not carried out, this can lead to recurrence of the trauma or the subject can be afflicted with reduced functional efficiency.

In rehabilitation of the ankle after an injury, one very important stage is the "proprioceptive" stimulation of the injured joint.

Proprioceptive receptors are specialised nerve receptors present in large numbers in the joints, mainly in ligaments and capsules.

In the foot, the proprioceptors are located in particular in the capsule and the ligaments of the ankle, subtalar and metatarsophalangeal joints of the great toe, notoriously recognised as fundamental areas for perfect dynamics in the upright position.

It is therefore fundamental for the rehabilitator to recover the proprioceptive abilities as quickly as possible and to stimulate them in order to restore full efficiency and functionality to the injured joint.

For proprioceptive rehabilitation, unstable surfaces are usually used, such as half-moon or half-sphere boards which consist of a support surface whose lower part is fitted with a half-moon, half-sphere or similar shaped element making the board unstable when stood on.

The main problem encountered with the use of known rehabilitation boards is that it is not possible to turn the half-moons of the board from a longitudinal position, with forward-backward obliqueness for flexion-extension movements, to a transverse position, with right-left obliqueness, for pronation-supination movements and inversion-eversion.

In effect, to change the orientation of the board it is necessary to physically rotate it by 90°, meaning that boards of this type must necessarily be square or circular so that the half-moon can be positioned longitudinally or transversely.

A square or circular shaped board, however, implies a limitation of the maximum angular excursions, due to the necessarily larger shape of the support surface.

### DESCRIPTION OF THE INVENTION

This invention proposes to provide a proprioceptive board for the rehabilitation of joints of both the upper and lower limbs, which can eliminate or at least reduce the drawbacks described above.

In particular the proprioceptive board for the rehabilitation of limbs and joints proposes to resolve the problems caused by the traditional impossibility of moving the half-moons from a longitudinal to a transverse position other than by rotating the entire board.

The invention also proposes to provide a proprioceptive board for the rehabilitation of limbs and joints that is easy to produce in order to be economically advantageous as well as extremely efficient from the point of view of versatility.

A further advantage proposed by the invention is to produce a proprioceptive board for rehabilitation that is equipped with means designed to visually control and if necessary record the information relative to the amplitude and the progress of the movements made during the exercising of the foot or the arm in the rehabilitation of the ankle and the shoulder respectively.

This is achieved by means of a proprioceptive board for rehabilitation of the joints according to the invention, with the features described in the main claim.

The dependent claims describe advantageous embodiments of the invention.

The proposed aims are achieved, according to the invention, by a proprioceptive board for rehabilitation of the joints which is equipped with a roller positioned underneath and in the centre of the support surface, the roller being fitted on a rotation surface which allows the roller to be moved from a substantially longitudinal position to a transverse position with respect to the board on which it is fitted.

The proprioceptive board for rehabilitation of the joints according to the invention is equipped with laser type orientation means which are positioned underneath the board and offer the possibility of displaying a trace of the movements being carried out.

This allows the rehabilitation subject to follow curves or indications of various types according to the different progress stages of the rehabilitation.

### DESCRIPTION OF THE DRAWINGS

Other features and advantages of the invention will become evident on reading the following description of one embodiment of the invention, provided as a non-binding example, with the help of the accompanying drawings in which:
- figure 1 is a schematic and prospective view of the overall board according to the invention, seen from above;
- figure 2 shows the underside of the board;

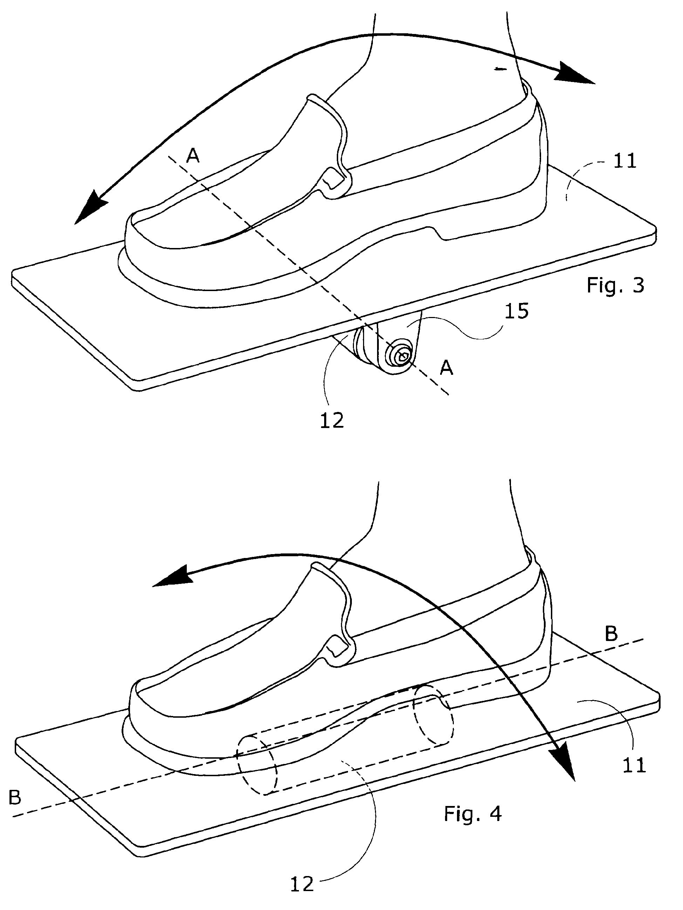

- figure 3 is a schematic and prospective view of the board during its use for exercise on the transverse axis for flexion-extension movements;
- figure 4 is a schematic and prospective view of the board during its use on the longitudinal axis for pronation-supination movements and inversion-eversion.

### DESCRIPTION OF ONE EMBODIMENT OF THE INVENTION

The proprioceptive board according to the invention for rehabilitation of the joints and upper and lower limbs, mainly although not exclusively used in the orthopedic sector for joint rehabilitation, is indicated overall with the number 10, and substantially consists of a support surface 11 which is rectangular or another similar elongated shape to allow the foot or arm to be rested on it, according to the limb to be rehabilitated.

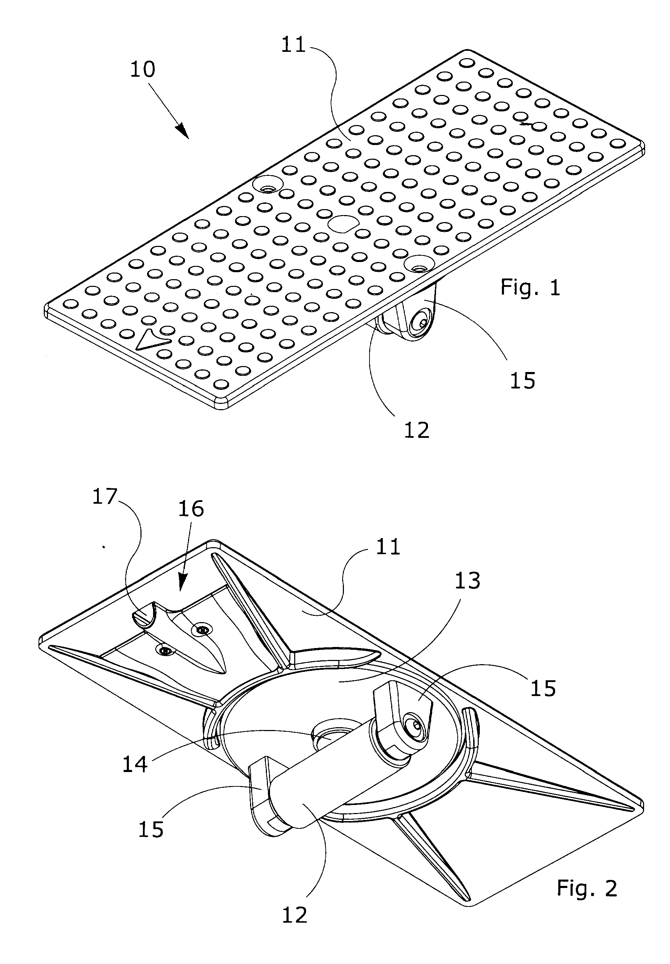

As can be clearly seen in figure 2, a roller 12 is fitted underneath the support surface 11, allowing the support surface to rock according to certain angulations.

The roller 12 is fitted to the lower side of the support surface by means of an interposed plate 13 hinged at the centre 14 to the centre of the board 11.

The roller 12 is positioned between two shoulders 15 positioned concentrically on the diameter of the plate 13, so as to be perfectly balanced with respect to the centre line of the plate and thus with respect to the centre line of the board.

This construction makes it possible to move the roller 12 from a transverse position to a longitudinal position and vice versa, with the possibility of also being placed in intermediate positions.

As can be imagined this allows the board to be used to carry out both flexion-extension movements on the transverse axis and pronation-supination and inversion-eversion on the longitudinal axis.

To angularly move the roller with respect to the board on which it is mounted, it is sufficient to turn the roller with its plate 13 to the angular position required, securing it in place with common screw-type fixing means, for example simply by moving the central rotation pin.

The rehabilitation board according to the invention is also equipped with an orientation aid 16 positioned on the lower part of the support surface.

The orientation aid consists of a light emission source, preferably the laser type, which projects its beam exactly in front of the board as an extension of the longitudinal centre line.

The light source emits its beam of light through a channel 17 positioned in the centre of the lower part of the board and on the shorter side.

The light emission source can direct beams of light that project against a wall in front of the board, and by using special graphs or curves drawn on work charts and positioned on the wall it is possible to copy the most appropriate movements for the rehabilitation of the joint to be treated.

To use the board, it is therefore sufficient to position the roller 12 transversally or longitudinally by turning the roller with its plate 13 until the required angular position is reached and securing it in the working position.

It is now possible to rest the foot or the arm or better the forearm or any other part of the upper or lower limbs, and to begin moving the foot, the leg and/or the wrist or the arm to catty out flexion-extension movements on the transverse axis, or pronation-supination movements and inversion-eversion on the longitudinal axis according to the angular position of the roller 12.

As can be noted, the board according to the invention is extremely practical and versatile to use, it being possible to move the lower roller to the required position and to follow the movements being carried out by means of the light beam projected on the wall.

The invention is described above with reference to a preferred embodiment. It is nevertheless clear that the invention is susceptible to numerous variations that lie within its scope within the framework of technical equivalents.

## Claims

1. A proprioceptive board (10) for the rehabilitation of joints, in particular for the treatment of the upper and/or lower limbs, the board consisting of a support surface (11) which is rectangular or another similar elongated shape to allow the part of the limb to be rehabilitated to be rested on it, **characterised in that** the lower side of the support surface (11) is fitted with a roller (12), which allows the support surface to rock according to certain angulations, the roller (12) being fitted on the lower side of the support surface by means of an interposed plate (13) hinged at its centre (14) to the centre of the board (11), and **in that** the board is equipped with an orientation aid (16) positioned on the lower side of the support surface.

2. A proprioceptive board (10) according to the foregoing claim, **characterised in that** the roller (12) is positioned between two shoulders (15) positioned concentrically on the diameter of the plate (13), in order to remain perfectly balanced with respect to the centre line of the plate and thus with respect to the centre line of the board.

3. A proprioceptive board (10) according to either of the foregoing claims, **characterised in that** the roller (12) can be moved from a transverse position to a longitudinal position and vice versa, with the possibility of also being placed in intermediate positions.

4. A proprioceptive board (10) according to any of the foregoing claims, **characterised in that** the orientation aid (16) consists of a light emission source, preferably the laser type, which projects its beam exactly in front of the board.

5. A proprioceptive board (10) according to any of the foregoing claim, **characterised in that** the orientation aid (16) projects its beam as an extension of the longitudinal centre line.

6. A proprioceptive board (10) according to any of the foregoing claim, **characterised in that** the orientation aid (16) emits its beam of light through a channel (17) positioned in the centre of the lower part of the board and on the shorter side.
